# EUROPEAN PATENT SPECIFICATION

(11) **EP 3 424 536 B1**
(45) Date of publication and mention of the grant of the patent: **27.01.2021**
(21) Application number: 17759312.6
(22) Date of filing: 27.02.2017
(51) Int. Cl.: A61K 31/085, A61P 23/00, A61K 9/08, A61K 9/51

(54) **ANAESTHETIC SOLUTION AND METHOD OF USE THEREOF FOR ANAESTHETISING, STUNNING AND SLAUGHTERING FISH**
ANÄSTHETISCHE LÖSUNG UND VERFAHREN ZUR VERWENDUNG DAVON ZUM ANÄSTHESIEREN, BETÄUBEN UND SCHLACHTEN VON FISCHEN
SOLUTION ANESTHÉSIANTE ET SON PROCÉDÉ D'APPLICATION POUR L'ANESTHÉSIE, L'ÉTOURDISSEMENT ET L'ABATTAGE DES POISSONS

(30) Priority: 04.03.2016 ES 201630258
(43) Date of publication of application: 09.01.2019
(73) Proprietor: Universidad Politecnica de Cartagena, 30202 Cartagena (Murcia) (ES)
(72) Inventor: GARCIA AYALA, Alfonsa, 30003 Murcia (ES); LOPEZ CÁNOVAS, Amanda Esperanza, 30003 Murcia (ES); LOPEZ GOMEZ, Antonio, 30202 Cartagena (Murcia) (ES); MULERO MENDEZ, Victoriano, Francisco, 30003 Murcia (ES); ROS CHUMILLAS, Maria, 30202 Cartagena (Murcia) (ES)
(74) Representative: Carpintero Lopez, Francisco
(86) International application number: PCT/ES2017/070108
(87) International publication number: WO 2017/149179

(56) References cited:
- WO-A1-01/70234
- WO-A1-98/54958
- NZ-A- 244 531
- Franziska Christine Oetinger: "Betäubung von Regenbogenforellen (Oncorhynchus mykiss) mit Nelkenöl und BHA - Stressbelastung und Produktqualität", , 18 July 2003 (2003-07-18), pages 1-84, XP055608466, Retrieved from the Internet: URL:https://edoc.ub.uni-muenchen.de/1496/1 /Oetinger_Franziska_C.pdf [retrieved on 2019-07-24]
- HERNANDEZ-SANCHEZ et al.: "Complexation of Eugenol (EG), as Main Component of Clove Oil and as Pure Compound, with - and HP- -CDs", Food and Nutrition Sciences, vol. 3, no. 6, 2012, pages 716-723, XP055414458,
- "AVMA Guidelines for Euthanasia of Animals", AVMA, 2013, XP055539861,
- Staudin-Al: "CAVAMAX® W7 FOOD", WACKER., 10 June 2014 (2014-06-10), XP055540143, [retrieved on 2017-05-29]

## Description

### Field of the Invention

The present invention belongs to the field of the technology for anesthetizing animals, particularly for stunning and slaughtering farmed fish and fish used in experiments, such as sea bream, sea bass, trout, salmon, and zebra fish, for example. More specifically, the present invention contemplates a new anesthetic solution for fish that is based on using essential oils encapsulated in β-cyclodextrins, formulated with ice or water. Likewise, it relates to a method for anesthetizing, stunning, and/or slaughtering farmed fish and fish used in experiments based on using said anesthetic solution.

### Background of the Invention

During their life cycle, farmed fish and fish used in experiments may be subjected to an entire series of handling situations which lead to stress and suffering, for example, when they are caught with dip nets and fish nets, while being weighed, classified, vaccinated, transported, and finally while being stunned and slaughtered. Fish used in experiments and research may also be subjected to surgical procedures that are more or less aggressive, such as when they are tagged, when sampling is performed, or when small incisions or more invasive interventions are carried out, for example.

In this sense, in the article *"*Scientific Opinion of the Panel on Animal Health and Welfare" published in the EFSA Journal, 2009, 1010, 1-52, the EFSA (European Food Safety Authority) also establishes as "Question No. EFSA-Q-2008-441" adopted on 20 March 2009, that the pre-slaughtering stages applied immediately before killing of the farmed fish have a direct and negative impact on fish welfare. For this reason, the EFSA highlights the need to develop methods suitable for being applied in those pre-slaughtering stages, and also during slaughtering, which prevent the fish from suffering too much stress. Additionally, in the mentioned Opinion EFSA stresses that all slaughtering methods used today in aquaculture on a commercial level keep the fish in a prolonged period of consciousness, usually of several minutes, such that the fish are subjected to high levels of stress and suffering.

As established by the authors Zahl *et al.* (Zahl I.H., Samuelsen O., Kiessling A. (2012). Anaesthesia of farmed fish: implications for welfare. Fish physiology and biochemistry, 38(1): 201-218), treatment with anesthetic agents may be of interest in all the situations mentioned above for the purpose of reducing suffering and assuring fish welfare.

In this sense, different anesthetic agents have been used in fish, where the most widely used agent is benzocaine (ethyl 4-aminobenzoate), metacaine, or tricaine methanesulfonate (MS-222) (ethyl 3-aminobenzoate), metomidate hydrochloride (methyl 3-(1-phenylethyl)imidazole-4-carboxylate hydrochloride), isoeugenol (2-methoxy-4-prop-1-enyl-phenol), 2-phenoxyethanol, and quinaldine (2-methylquinoline). Like eugenol, isoeugenol is one of the constituents of clove essential oil, which is another widely used fish anesthetic.

The protocols for anesthetizing fish usually involve the use of only one of these anesthetic agents, but they can be used in combination with analgesics. The anesthetic agent is usually dispersed or dissolved in water and the fish absorbs it mainly through its gills, even though small amounts can also be absorbed through its skin (*Zahl et al. 2012*). The anesthetizing effect is generally evaluated by means of induction and recovery time, reflex reactions to external stimuli, and responsiveness to handling.

According to the preceding article by *Zahl et al.* (2012), the progress of induction and the depth of anesthesia are generally divided into distinct stages and "planes" (see Table 1 of this article by *Zahl et al. 2012*) which can be characterized in animals by means of known clinical indicators (behavior, activity, corneal reflexes and pupil size, muscle tone, reflexes, respiratory rate, heart rate, and blood pressure). However, since some of these indicators are difficult to evaluate in fish, it may be very difficult to distinguish the aforementioned stages and "planes", particularly in situations in which induction is rapid. For this reason, these stages are generally described by means of changes in swimming activity, balance, respiratory rate, and reactions to external stimuli (see Table 1 of the article by *Zahl et al. 2012* on the stages of anesthesia in fish). Therefore, in most of the comparative studies or evaluations of anesthetics in fish, the fish are anesthetized until reaching a specific stage which usually corresponds with a loss of balance, absence of swimming activity, lack of response to external stimuli, and decreased or even stopped respiration (which as mentioned in Table 1 of the mentioned article *by* Zahl *et al. (2012)* would correspond with stage III, plane 3, even approaching stage IV) (Hoskonen P., Pirhonen J. (2004). Temperature effects on anaesthesia with clove oil in six temperate-zone fishes. J Fish Biol 64:1136-1142*;* Mattson N.S., Riple T.H. (1989). Metomidate, a better anesthetic for cod (Gadus morhua) in comparison with benzocaine, MS-222, chlorobutanol, and phenoxyethanol. Aquaculture 83:89-94; Mylonas C.C., Cardinaletti G., Sigelaki I., Polzonetti-Magni A. (2005). Comparative efficacy of clove oil and 2-phenoxyethanol as anesthetics in the aquaculture of European sea bass (Dicentrarchus labrax) and gilthead sea bream (Sparus aurata) at different temperatures. Aquaculture 246:467-481*;* Stehly G.R., Gingerich W.H. (1999). Evaluation of AQUI-S (TM) (efficacy and minimum toxic concentration) as a fish anaesthetic sedative for public aquaculture in the United States. Aquaculture Research 30:365-372*;* Sylvester J.R., Holland L.E. (1982). Influence of temperature, water hardness, and stocking density on MS-222 response in three species of fish. Progress Fish Cultur 44:138-141*).*

NZ 244531 describes a solution of eugenol or isoeugenol for sedating, anaesthetising or euthanising aquatic organisms.

As inferred from the different research papers mentioned above, the fish must remain in contact with the mentioned anesthetic agents usually for several minutes (2 to 15 minutes) in order to achieve a suitable anesthetizing effect (see Table 2 of the article by Zahl *et al. 2012* concerning recommended doses and exposure times for different fish anesthetic agents). This will have an influence on the stress suffered by the fish and on the quality of the end product obtained from farmed fish. In fact, the post-mortem blood and muscle biochemistry and the start of rigor mortis are influenced by the method used in the pre-slaughtering handling, stunning, and slaughtering of fish which may in turn negatively affect the organoleptic and market qualities of the end product, such as the filleting of the corresponding farmed fish *(*Zampacavallo G., Parisi G., Mecatti M., Lupi P., Giorgi G., Poly B.M. (2015). Evaluation of different methods of stunning/killing sea bass (Dicentrarchus labrax) by tissue stress/quality indicators. Journal of food science and technology, 52(5):2585-2597).

On the other hand, although the guidelines for ecological (also referred to as organic) aquaculture animal production (see, for example, Commission Regulation (EC) No. 710/2009, of 5 August 2009) do not indicate the specific techniques that must be used, they do establish that the slaughtering techniques shall render fish immediately unconscious and insensible to pain. This can be interpreted to mean that those techniques in which fish are anesthetized (or stunned) and slaughtered in the shortest time possible, causing the lowest amount of stress and suffering possible, are desired. However, the most advanced techniques for stunning/slaughtering fish fail to achieve shorter times for these operations, so the level of stress produced is relatively high. This leads to a relatively shorter shelf life in the preservation of fish stunned/slaughtered with these techniques, as clearly shown in the paper by Zampacavallo *et al.* (2015).

In a study relating to this topic, as described in the article by Lerfall *et al.* (Lerfall J., Roth B., Skare E.F., Henriksen A., Betten T., Dziatkowiak-Stefaniak M.A., Rotabakk B. T. (2015). Pre-mortem stress and the subsequent effect on flesh quality of pre-rigor filleted Atlantic salmon (Salmo salar L.) during ice storage. Food Chemistry, 175:157-165), excessive stress results have been obtained when stunning is performed with a seawater and crushed ice mixture (with a weight ratio of 1:1, and at a temperature of about -0.3°C). Said authors (*Lerfall et al., 2015*) have clearly shown that plasma and tissue metabolite levels are affected by the stress induced during stunning and slaughtering, which translates into a significant increase in blood glucose levels.

Furthermore, interest in avoiding chemical synthesis agents both during production and during stunning and slaughtering can be inferred from the philosophy of ecological food production in general (also referred to as organic production, including organic agriculture and aquaculture). Therefore, clove essential oil (CEO) would constitute a desirable anesthetic in organic or ecological aquaculture. However, the problem with using this anesthetic is its lipophilic character (it is an oil) and poor dissolution in water. This leads to the need to use high doses of this anesthetic in order to have an effect in a relatively short time. For example, for stunning/slaughtering sea bass, a dose of 105 mg/L of water in the bath used for stunning this fish is proposed in the research paper published by Simitzis *et al. (*Simitzis P.E., Tsopelakos A., Charismiadou M.A., Batzina A., Deligeorgis S.G., Milliou, H. (2014). Comparison of the effects of six stunning/killing procedures on flesh quality of sea bass (Dicentrarchus labrax, Linnaeus 1758) and evaluation of clove oil anaesthesia followed by chilling on ice/water slurry for potential implementation in aquaculture. Aquaculture Research, 45(11):1759-1770*).*

To increase water solubility of clove essential oil and the absorption thereof by fish, and to thereby improve its anesthetizing effect, this essential oil could be used in combination with cyclodextrins, forming an inclusion complex. The inclusion of this clove essential oil in β-cyclodextrins (β-CD) has already been studied, but its effect or interest in anesthetizing or slaughtering fish has never been tested.

However, the use of anesthetic agents for human beings in combination with β-cyclodextrins, forming inclusion complexes, has been proposed. In this sense, patent US 8,975,245 B2 (2015) entitled *"Anaesthetic formulation",* clearly shows the interest in using neuroactive steroid anesthetic agents in combination with cyclodextrins, forming inclusion complexes, but only for use in human beings, and it does not analyze the interest in using the mentioned clove essential oil in combination with β-cyclodextrins for use in anesthetizing fish.

The paper by De Oliveira *et al.* (De Oliveira G.B., Guimarães A.G., Araújo A.S., Quintans S.S., Santos M.R.V., Quintans-Júnior L.J. (2015). Cyclodextrins: improving the therapeutic response of analgesic medications: a patent review. Expert opinion on therapeutic patents, 25(8):897-907) clearly shows that cyclodextrins can be found in at least 35 pharmaceutical products as anti-cancer agents or as analgesic and anti-inflammatory medications to be applied in human beings. This corresponds with the results of several studies which have demonstrated that medications in the form of inclusion complexes with cyclodextrins may be beneficial in human beings in terms of solubility, stability, and also in terms of improving pharmacological response in comparison with the medication alone.

The formation of inclusion complexes between cyclodextrins and different molecules has been widely studied in the literature. In this manner, the behavior of these molecules is successfully changed, making them more water-soluble, for example. Cyclodextrins constitute a group of natural cyclic oligosaccharides derived from starch with six, seven, or eight glucose molecules bound by α(1-4) glycosidic bonds forming a cylindrical-shaped structure referred to as α-, β-, and γ-cyclodextrins, respectively. The central cavity of these molecules is hydrophobic, whereas the outer edges of the walls surrounding this cavity are hydrophilic. This hydrophobic cavity forms inclusion complexes with a wide range of organic host molecules. As establish in the paper by Marques *(*Marques H.M.C. (2010). A review on cyclodextrin encapsulation of essential oils and volatiles. Flavour and Fragrance Journal, 25(5):313-326*),* cyclodextrins can be considered nano-encapsulation agents and the formation of the cyclodextrin-host complex is equivalent to a molecular encapsulation, given that the host molecules are isolated from one another and dispersed at the molecular level in an oligosaccharide matrix.

Inclusion complexes of essential oils with cyclodextrins *(Marques 2010)* can also be formed. For example, the paper by Hernandez-Sanchez *et al. (*Hernández-Sanchez P., López-Miranda S., Lucas-Abellan C., Núñez-Delicado E. (2012). Complexation of Eugenol (EG), as Main Component of Clove Oil and as Pure Compound, with β- and HP-β-CDs. Food and Nutrition Sciences, 3:716-723*),* studies the molecular association between β-CD, eugenol, and clove essential oil, obtaining complexes that are water-insoluble and not suitable for being applied in anesthetizing fish.

In view of the needs of the state of the art in relation to the problems derived from anesthetizing, stunning, and slaughtering fish, the authors of the present invention have developed a new formulation based on using natural anesthetics nanoencapsulated in β-cyclodextrins, giving rise to water-soluble inclusion complexes. The inclusion complexes thus formed are formulated with water and/or ice, which surprisingly gives rise to solutions that have a very low concentration of anesthetic agent in comparison with those used in the state of the art and equally effective. These solutions applied to anesthetizing, stunning, and slaughtering farmed fish or fish used in experiments therefore provide very significant advantages with respect to the state of the art given that, in addition to reducing the amount of anesthetic agent, and therefore the unwanted effects derived from it, it allows significantly reducing animal suffering because the animal is stunned or loses consciousness in a matter of a few seconds, and slaughtering is carried out with much less suffering in comparison with the current methods.

### Brief Description of the Drawings

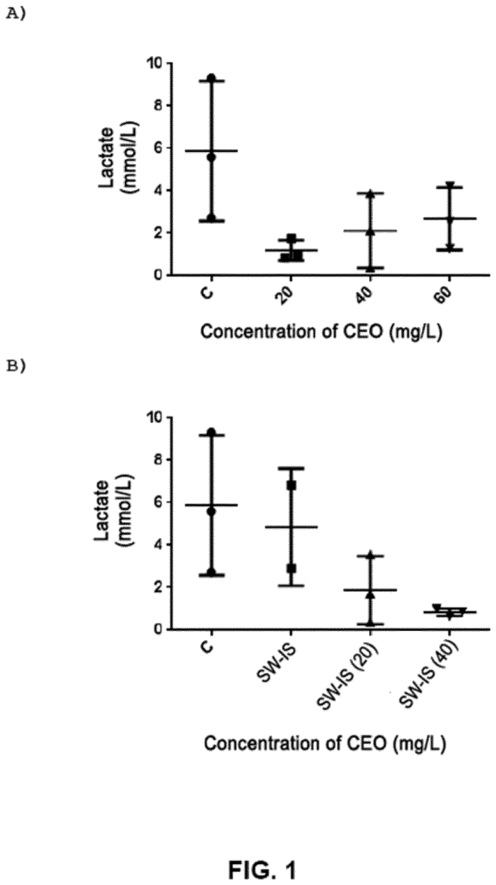

Figure 1 shows blood lactate levels of sea bream (mmol/L) depending on the type of stunning applied: A) seawater at room temperature (28-29°C) with CEO+β-CD at different concentrations (20, 40, and 60 mg/L); B) seawater ice slurry with CEO+β-CD at different concentrations at -5°C. C = crushed ice and seawater (1:1 by weight) at -0.5°C, without CEO; SW-IS = seawater ice slurry (without CEO); SW-IS (20, 40) = seawater ice slurry with CEO+β-CD (20, 40 mg/L).

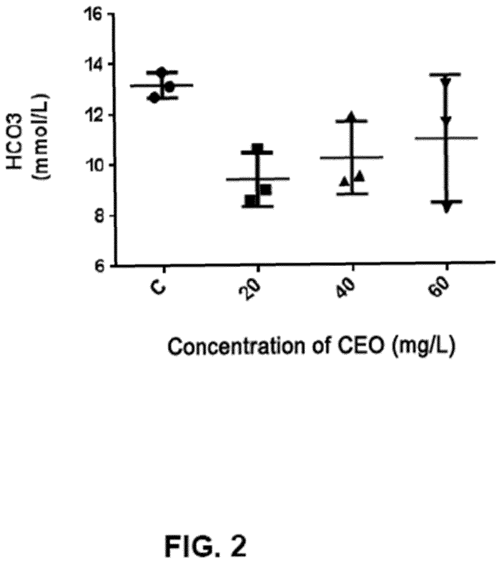

Figure 2 shows blood HCO₃ levels of sea bream (mmol/L) depending on the type of stunning applied. Results are shown for treatments with seawater at room temperature (28-29°C) with different concentrations of CEO+β-CD (20, 40, and 60 mg/L); C = crushed ice and seawater (1:1 by weight) at -0.5°C, without CEO.

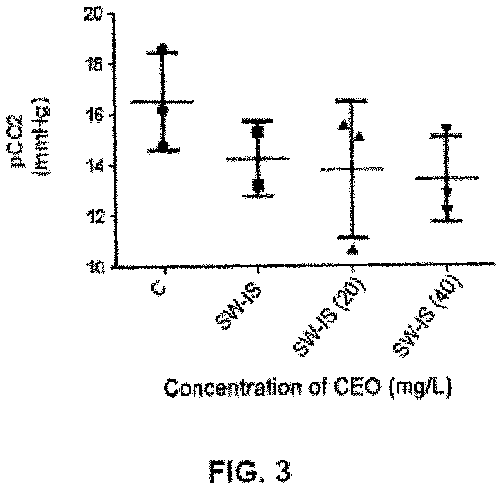

Figure 3 shows blood pCO₂ levels of sea bream (mmHg) depending on the type of stunning applied. C = crushed ice and seawater (1:1 by weight) at -0.5°C; SW-IS = seawater ice slurry, at -5°C; SW-IS (20, 40) = seawater ice slurry with CEO+β-CD (20 or 40 mg/L).

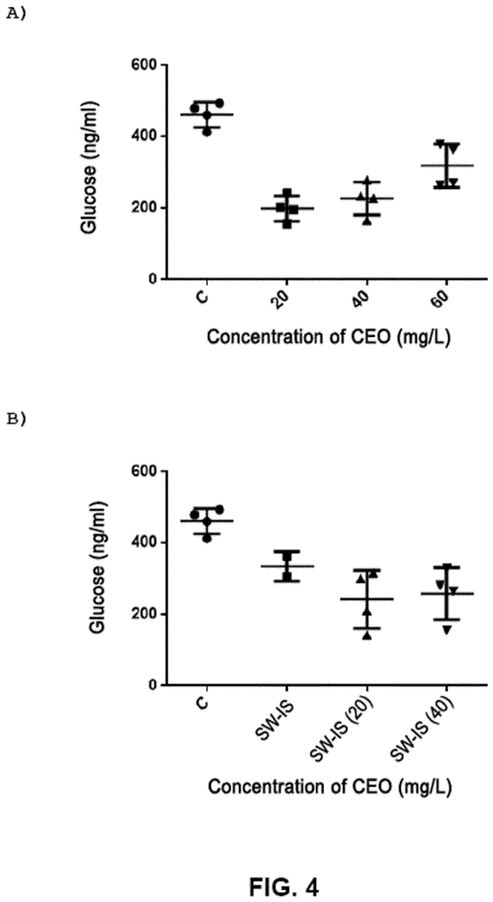

Figure 4 shows serum glucose levels of sea bream (ng/ml) depending on the type of stunning applied: A) seawater at room temperature (28-29°C) with CEO+β-CD at different concentrations (20, 40, and 60 mg/L); C = crushed ice and seawater with CEO+β-CD (1:1 by weight) at -0.5°C, without CEO; B) seawater ice slurry with
   CEO+β-CD at different concentrations and at -5°C; SW-IS (0, 20, 40) = seawater ice slurry with CEO+β-CD (0, 20, or 40 mg/L).

### Detailed Description of the Invention

The present invention relates to a new solution of a natural anesthetic, based on clove essential oil (CEO) and β-cyclodextrins (β-CD) having a moisture content between 1 and 4%, in combination with water, water with crushed ice, or ice slurry, for anesthetizing, stunning, and slaughtering farmed fish and fish used in experiments, such as sea bream, sea bass, trout, salmon, and zebra fish, for example.

A main embodiment of the invention contemplates an anesthetic solution (hereinafter, the solution of the invention) comprising a natural anesthetic agent nanoencapsulated in β-cyclodextrins, forming a water-soluble inclusion complex. The proportion of the natural anesthetic in the inclusion complex is comprised between 5 and 30% of the total mass of the complex, preferably between 10 and 20%, and the proportion of β-cyclodextrins in the inclusion complex is comprised between 70 and 95% of the total mass of the complex, preferably between 80 and 90%.

The anesthetic agent is formulated in freshwater, in seawater, in ice slurry (ice slurry being defined as established in the article by Kauffeld, M., Wang, M. J., Goldstein, V., Kasza, K. E. (2010). Ice slurry applications. International Journal of Refrigeration, 33(8), 1491-1505), or in water with crushed ice, at a concentration comprised between 5 and 60 mg/kg, preferably between 10 and 40 mg/kg, depending on the temperature of the water (concentrations equivalent to mg of anesthetic agent per kg of initial water).

The natural anesthetic agent used in the solution of the invention is an essential oil, particularly clove essential oil, and/or the components thereof, such as eugenol and isoeugenol.

β-cyclodextrins used for formulating the anesthetic object of this invention must be food-grade β-cyclodextrins that are classified as a food additive referred to as E-459. They must have the appearance of a white odorless powder and have a minimum purity of 98% (on an anhydrous basis), a moisture content not greater than 14% (determined using the Karl Fischer method), preferably between 1 and 4%, and a content of other cyclodextrins not greater than 2% (on an anhydrous basis). They must be colorless when dissolved in water.

The moisture content of the β-cyclodextrins used in the formulation of less than 14% significantly improves the water solubility of the corresponding inclusion complex that is formed. In the moisture range of 1-4%, the solubility of the complex is complete, with complete absence of precipitates or suspended particles in the solution.

The essential oil can nanoencapsulated in β-cyclodextrins by means of any of the following methods used in the state of the art: kneading method, coprecipitation method (based on phase solubility or not), method of heating in a sealed container or recipient, gas (or vapor)-liquid interaction method, lyophilization method, atomization method, or by means of using supercritical fluid technology (as described in *Marques et al., 2010*).

In particular embodiments of the solution of the invention, the anesthetic agent is formulated in ice slurry, where the ice is in a proportion comprised between 20 and 70% by weight with respect to the total weight of the water and ice mixture.

In another particular embodiment of the solution of the invention, the anesthetic agent is formulated in water with crushed ice, the water:crushed ice proportion by weight being comprised between 1:1, 1:2, and 1:3, or in a proportion that is in between same.

Another main embodiment of the invention contemplates the use of the solution of the invention for anesthetizing, stunning, and/or slaughtering farmed fish and fish used in experiments. The use of the solution in these applications allows significantly reducing animal suffering experienced by this fish because the animal is stunned or loses consciousness in a matter of a few seconds, and slaughtering is carried out with much less animal suffering.

Based on these applications, another main embodiment of the invention contemplates a method for anesthetizing farmed fish and fish used in experiments. Said method comprises the following steps:
- first preparing the anesthetic solution contemplated in the invention formulated in freshwater at a temperature comprised between 0 and 30°C, in seawater at a temperature comprised between 0 and 30°C, in ice slurry at a temperature comprised between -8 and 0°C, or in water with crushed ice at a temperature comprised between -1 and 0°C,
- then submerging the fish caught by means of dip nets or by means of pumping in one of the prepared solutions, introducing, at most, as many kilograms of fish as kilograms of solution,
- keeping the fish submerged in the solution for a time comprised between 5 and 55 seconds, depending on the size and type of fish, and on the type of solution used, until the fish show signs of being anesthetized, such as a loss of balance, absence of swimming activity, and lack of response to external stimuli,
- finally, removing the fish from the anesthetizing solution.

Another main embodiment of the invention contemplates a method for stunning and slaughtering fish comprising the following steps:
- first preparing the anesthetic solution of the invention formulated in ice slurry at a temperature comprised between -8 and 0°C, or in water with crushed ice at a temperature comprised between -1 and 0°C,
- then submerging the fish caught by means of dip nets or by means of pumping in the obtained solution, introducing, at most, as many kilograms of fish as kilograms of prepared solution for stunning the fish,
- keeping the fish for 5-55 seconds, depending on the size and type of fish, and on the type of solution used, until the fish show signs of being stunned or anesthetized, such as a loss of balance, absence of swimming activity, and lack of response to external stimuli, and
- finally, removing the fish from the stunning solution and introducing them in water with crushed ice in order to slaughter them by hypothermia (in a water:ice proportion by weight of 1:1, 1:2, 1:3, or in a proportion that is in between same), and at a temperature comprised between -1 and 0°C, depending on if the method uses seawater and crushed ice or freshwater and crushed ice or ice slurry, where the ice is in a proportion comprised between 20 and 70% by weight with respect to the total weight of the water and ice mixture, at a temperature between -8 and 0°C. The fish are introduced, at most, in a proportion of as many kilograms of fish as kilograms of solution (water and crushed ice or ice slurry) until they are slaughtered. Once slaughtered, the fish are kept in the water with ice.

Several examples are listed below as non-exclusive embodiments and applications of the invention which clearly shown that stunning or anesthetizing times are considerably reduced, and therefore animal suffering while stunning and slaughtering the farmed fish or fish used in experiments is thereby reduced.

### Examples

### Example 1

### Material and methodology followed

To carry out the different practical embodiments of the invention, different anesthetizing or stunning treatments were applied to a total of 30 farmed sea breams with a mean weight of 465.75 ± 102.12 g. Once stunned, the fish were then slaughtered in seawater with crushed ice (in a proportion by weight of 1:1).

This example studied the anesthetizing effect of different concentrations of clove essential oil (CEO) included in β-CD (20, 40, and 60 mg/L) formulated:
- in seawater at room temperature (28-29°C),
- in seawater ice slurry at -5°C (60% of ice), or
- in crushed ice plus seawater (1:1) with a concentration of 20 mg/L of CEO included in β-CD and at a temperature of -0.5°C.

The control treatment used (referred to as treatment C in Figures 1-4) reproduces the technique used today in industrial aquaculture in which stunning and slaughtering are performed by hypothermia in seawater with crushed ice (in a proportion by weight of 1:1). Furthermore, stunning in ice slurry at -5°C without clove essential oil included in β-CD was also analyzed.

The nanoencapsulation or preparation of the corresponding inclusion complex (such as a solid complex) according to a formulation or proportion of clove essential oil to β-CD of 14.28%/85.72% was carried out by means of the kneading method (as described in detail in *Marques, 2010*).

A total of 4 fish were analyzed in each treatment, where the visual parameters of fish behavior and analytical parameters in response to stunning are analyzed. After stunning (characterized by the fish showing signs of being anesthetized, such as a loss of balance, absence of swimming activity, and lack of response to external stimuli), blood samples were drawn from the tail vein, using lithium heparin as a anticoagulant, immediately after stunning or anesthetizing each individual. An analysis was performed using disposable CG4 cartridges and an i-STAT blood analyzer, measuring lactate, pCO₂ (partial blood CO₂ pressure), HCO₃, and glucose according to the method followed by Lerfall *et al.* (2015).

All the aforementioned treatments, experiments, and analyses performed on fish were carried out according to the approval of the Ethics Committee of the Universidad de Murcia.

### Obtained results

In terms of blood lactate levels (mmol/L) (Figure 1), significant differences were observed between the fish treated with crushed ice and seawater (1:1) and seawater at 28-29°C at different concentrations of CEO+β-CD (Figure 1A). Higher lactate values are obtained in those individuals stunned with crushed ice and seawater without anesthetic (9.31-5.60 mmol/l) in comparison with those treated in seawater with different concentrations of CEO+β-CD (1.74-1 mmol/l, 3.88-0.36 mmol/l and 4.22-1.28 mmol/l in 20, 40, and 60 mg/L of CEO+β-CD). Significant differences were also seen between individuals stunned at different concentrations of CEO+β-CD in ice slurry at -5°C (Figure 1B). In this sense, higher lactate values were found in individuals that had been stunned with crushed ice and seawater (1:1) without an anesthetic agent. It was therefore demonstrated that very low stress levels are achieved when fish are stunned in seawater ice slurry with anesthetic concentrations of 40 mg/L (0.99-0.64 mmol/l).

When the results of blood HCO₃ (mmol/L) (Figure 2) were analyzed, significantly higher values were observed in fish stunned with crushed ice and seawater (1:1) when compared to those individuals stunned with the anesthetic at concentrations of 20 and 40 mg/l and at room temperature.

As regards blood pCO₂ values (mmHg) (Figure 3), differences were observed in the mean values obtained for those fish stunned with concentrations of CEO+β-CD of 40 mg/L and in ice slurry at -5°C if compared with the conventional stunning and slaughtering method which uses crushed ice in seawater (1:1) without CEO (Treatment C).

When comparing serum glucose levels (ng/mL) of each individual sea bream (Figure 4A) treated with CEO+β-CD at different concentrations with seawater at room temperature (28-29°C), no significant differences were observed between them (Figure 4A). Nevertheless, a certain trend of the glucose level increasing as the applied dose of anesthetic increases was observed (198.25 ng/mL to 20 mg/L of clove oil; 226 ng/mL to 40 mg/L of clove oil; and 318.5 ng/mL to 60 mg/L of clove oil). In any case, the blood glucose level in the sea bream is always lower when the anesthetic solution object of this patent is used, if compared with stunning with seawater with crushed ice (1:1). Based on the foregoing, and in this case, the invention proposes using the lowest concentration of CEO+β-CD (20 mg/L) when sea bream is stunned in seawater at room temperature. This would be a method of interest for anesthetizing farmed fish or fish used in experiments when slaughtering of the fish is not desired. On the other hand, significant differences are seen between the individuals stunned with ice slurry at -5°C (Figure 4B) with and without CEO+β-CD, and the fish stunned with crushed ice and seawater (1:1) without CEO+β-CD. The fish stunned with ice slurry with CEO+β-CD suffer less stress than the fish stunned in seawater and crushed ice (1:1).

In these embodiments of the invention, significantly higher lactate, pCO₂, HCO₃, and glucose levels were observed in sea bream specimens stunned with seawater and crushed ice (1:1) compared to those stunned with the anesthetic based on CEO included in β-CD in ice slurry at -5°C or at room temperature. The lower values of these parameters, which are indicators of stress in fish during stunning, correspond with the fact that when the stunning and slaughtering technology object of this patent is used on farmed sea bream, stunning times of 10-15 s (in ice slurry with CEO+ β-CD) or of less than 55 seconds (in seawater with crushed ice, with CEO+β-CD) are achieved compared to times of more than 4 minutes obtained with seawater and crushed ice (1:1) without CEO+β-CD (which is the conventional stunning and slaughtering method in aquaculture).

Similar results of stress when stunning is performed with seawater and crushed ice have been obtained in studies by other authors (such as *Lerfall et al. 2015*). These authors found that tissue and plasma metabolite levels are altered by the stress induced in salmon slaughtered with seawater and crushed ice (1:1), which translates, for example, into a significant increase in blood glucose levels and into higher blood lactate levels. The effects of stress observed by *Lerfall et al. (2015)* in salmon also link induced hypercapnia with high pCO₂ and HCO₃ levels in specimens stunned and slaughtered with the conventional stunning and slaughtering method which uses seawater and crushed ice.

With these embodiments of the formulation and methods of application of the new natural anesthetic solution object of this patent, it is demonstrated that much shorter stunning times (from 10 to 55 seconds) are achieved in comparison with the current conventional method used in aquaculture (water with crushed ice in a proportion by weight of 1:1). Therefore, this natural anesthetic solution and the method of application thereof for stunning and slaughtering fish also leads to less suffering and stress in the fish, so it also contributes to a significant improvement in animal welfare.

### Example 2

### Solubility tests

To carry out these solubility tests, aqueous solutions were prepared with three different concentrations of clove essential oil (5, 10, and 15 mg/L) forming inclusion complexes with two types of β-CDs (non-modified native β-CD and food-grade β-CD) which are characterized, among other things, by having a different moisture content:
A. Commercial β-CDs with a moisture content of 14.5%
B. β-CDs of the invention with a moisture content of 4%

β-CDs (B) with 4% moisture have a more powdery texture than β-CDs with 14.5% moisture (A), the latter having a more granular texture.

The inclusion complex of the clove essential oil with β-CDs was prepared following the kneading method indicated by *H.M.C. Marques [*Marques H.M.C., 2010, "A review on cyclodextrin encapsulation of essential oils and volatiles", Flavour and Fragrance Journal, 25(5), 313-326*].* The inclusion complex was obtained by incorporating the clove essential oil (CEO) to the β-CDs according to an equimolecular ratio. A weight:weight ratio of 1 g of CEO for every 6.90 g of β-CDs was used. Solubility tests were performed for these complexes in 1 L of water using the following amounts of the inclusion complex formed:
- the dissolution of 39.5 mg of β-CD+CEO complex was tested for 5 mg/L of CEO in 1 L of water,
- the dissolution of 79 mg of β-CD+CEO complex was tested for 10 mg/L of CEO in 1 L of water, and
- the dissolution of 118.5 mg of β-CD+CEO complex was tested for 15 mg/L of CEO in 1 L of water.

The solubility tests were performed in a laboratory at room temperature (20°C) using flasks with 1 L of water, adding the aforementioned amounts of inclusion complexes obtained from CEO with β-CDs (A and B), and with continuous magnetic stirring. The complexes were dissolved at the same time in different flasks, maintaining stirring for a time of 2 hours once they were added to each flask with water.

Regardless of the concentration of CEO that has been established, the obtained solubility results were dependent on the amount of initial moisture of the β-CDs used.

After the first 30 minutes, a better dissolution of the β-CD+CEO inclusion complex with the β-CDs of the invention (B) with respect to commercial β-CDs (A) can grossly be observed. After 2 hours, a complete dissolution of the β-CD+CEO complex which uses β-CDs with a maximum moisture content of 4% (B) was grossly observed, whereas small suspended particles were observed in the flasks with the complexes formed with β-CDs with 14.5% moisture (A). When the stirring was stopped and the flasks were left to stand for 5 hours, it can be observed that the solutions with the complexes with β-CDs with 14.5% moisture contained precipitates at the base of the flask, showing a clear insolubility of the complex for the three concentrations of clove essential oil that were studied. Surprisingly, the solution containing the β-CD+CEO inclusion complex formed with β-CDs with 4% moisture (B) did not contain any precipitate or suspended particles at the bottom of the flask, which is indicative of a suitable solubility for the three concentrations of clove essential oil that were tested (5, 10, and 15 mg/L), forming inclusion complexes with the β-CDs.

## Claims

1. Anesthetic solution comprising:
- a natural anesthetic agent selected from clove essential oil, isoeugenol, and/or eugenol,
- food-grade β-cyclodextrins forming a water-soluble inclusion complex with the natural anesthetic agent, and
- water selected from freshwater, seawater, ice slurry, or water with crushed ice,
**characterized in that** the proportion of the natural anesthetic in the inclusion complex is comprised between 5 and 30% of the total mass of the complex, the proportion of β-cyclodextrins in the inclusion complex is comprised between 70 and 95% of the total mass of the complex, the anesthetic agent is at a concentration comprised between 5 and 60 mg of anesthetic agent/kg of water, and **in that** the β-cyclodextrins used for formulating the anesthetic solution have a moisture content between 1 and 4%.

2. Anesthetic solution according to claim 1, wherein the proportion of the anesthetic agent in the inclusion complex is comprised between 10 and 20%, and the proportion of β-cyclodextrins is comprised between 80 and 90%.

3. Anesthetic solution according to any of claims 1 or 2, wherein the anesthetic agent is at a concentration comprised between 10 and 40 mg of anesthetic agent/kg of water.

4. Anesthetic solution according to any of claims 1 to 3, **characterized in that** the water used in the solution is ice slurry, where the ice is in a proportion comprised between 20 and 70% by weight with respect to the total weight of the water and ice mixture.

5. Anesthetic solution according to any of claims 1 to 3, **characterized in that** the water used in the solution is water with crushed ice, where the water: ice proportion by weight is comprised between 1:1 and 1:3.

6. Use of an anesthetic solution according to claims 1 to 5 for slaughtering fish.

7. Method for slaughtering fish comprising the following steps:
i) preparing an anesthetic solution according to claims 1 to 5, formulated in ice slurry at a temperature comprised between -8°C and 0°C, or in water with crushed ice at a temperature comprised between -1°C and 0°C,
ii) submerging in the solution prepared in i), at most, as many kilograms of fish as kilograms of solution,
iii) keeping the fish submerged in the solution prepared in i) for a time comprised between 5 and 55 seconds, until the fish are stunned,
iv) removing the stunned fish from the solution,
v) introducing the stunned fish in water with ice in a water: ice proportion by weight comprised between 1:1 and 1:3 and at a temperature comprised between -1°C and 0°C in a proportion, at most, of as many kilograms of fish as kilograms of water with ice, until the fish are slaughtered.

## Patentansprüche

1. Anästhetische Lösung, umfassend:
- ein natürliches Anästhetikum, ausgewählt aus ätherischem Nelkenöl, Isoeugenol und/oder Eugenol,
- β-Cyclodextrine mit Lebensmittelqualität, die mit dem natürlichen Anästhetikum einen wasserlöslichen Einschlusskomplex bilden, und
- Wasser, ausgewählt aus Frischwasser, Meerwasser, Eis-Wasser-Gemisch oder Wasser mit zerkleinertem Eis, **dadurch gekennzeichnet, dass** der Verhältnisanteil des natürlichen Anästhetikums in dem Einschlusskomplex zwischen 5 und 30% der Gesamtmasse des Komplexes liegt, der Verhältnisanteil von β-Cyclodextrinen in dem Einschlusskomplex zwischen 70 und 95% der Gesamtmasse des Komplexes liegt, das Anästhetikum eine Konzentration hat, die zwischen 5 und 60 mg Anästhetikum/kg Wasser liegt, und dadurch, dass die β-Cyclodextrine, die zur Formulierung der anästhetischen Lösung verwendet werden, einen Feuchtigkeitsgehalt zwischen 1 und 4% haben.

2. Anästhetische Lösung nach Anspruch 1, wo der Verhältnisanteil des Anästhetikums in dem Einschlusskomplex zwischen 10 und 20% liegt und der Verhältnisanteil von β-Cyclodextrinen zwischen 80 und 90% liegt.

3. Anästhetische Lösung nach einem der Ansprüche 1 oder 2, wobei das Anästhetikum eine Konzentration hat, die zwischen 10 und 40 mg Anästhetikum/kg Wasser liegt.

4. Anästhetische Lösung nach einem der Ansprüche 1 bis 3, wobei das in der Lösung verwendete Wasser Eis-Wasser-Gemisch ist, wobei das Eis in einem Verhältnisanteil, der zwischen 20 und 70 Gew.-% liegt, bezogen auf das Gesamtgewicht des Wasser-und-Eis-Gemisches, vorhanden ist.

5. Anästhetische Lösung nach einem der Ansprüche 1 bis 3, **dadurch gekennzeichnet, dass** das in der Lösung verwendete Wasser Wasser mit zerkleinertem Eis ist, wobei das Gewichtsverhältnis Wasser:Eis zwischen 1:1 und 1:3 liegt.

6. Verwendung einer anästhetischen Lösung nach Ansprüchen 1 bis 5 zum Schlachten von Fischen.

7. Verfahren zum Schlachten von Fischen, umfassend die folgenden Schritte:
i) Herstellen einer anästhetischen Lösung nach Ansprüchen 1 bis 5, formuliert in einem Eis-Wasser-Gemisch bei einer Temperatur zwischen -8°C und 0°C oder in Wasser mit zerkleinertem Eis bei einer Temperatur zwischen -1°C und 0°C,
ii) Eintauchen in die in i) hergestellte Lösung höchstens so viele Kilogramm Fische wie Kilogramm Lösung,
iii) Eingetauchthalten der Fische in der in i) hergestellten Lösung für eine Zeit, die zwischen 5 und 55 Sekunden liegt, bis die Fische betäubt sind,
iv) Entfernen der betäubten Fische aus der Lösung,
v) Einführen der betäubten Fische in Wasser mit Eis in einem Gewichtsverhältnis Wasser:Eis, das zwischen 1:1 und 1:3 liegt, und bei einer Temperatur zwischen -1°C und 0°C in einem Verhältnisanteil von höchstens so vielen Kilogramm Fische wie Kilogramm Wasser mit Eis, bis die Fische geschlachtet werden.

## Revendications

1. Solution anesthésique comprenant :
- un agent anesthésique naturel choisi parmi l'huile essentielle de clou de girofle, l'isoeugénol et/ou l'eugénol,
- des β-cyclodextrines de qualité alimentaire formant un complexe d'inclusion hydrosoluble avec l'agent anesthésique naturel, et
- une eau choisie parmi l'eau douce, l'eau de mer, la boue glacée ou l'eau avec de la glace pilée,
**caractérisée en ce que** la proportion de l'anesthésique naturel dans le complexe d'inclusion est comprise entre 5 et 30% de la masse totale du complexe, la proportion de β-cyclodextrines dans le complexe d'inclusion est comprise entre 70 et 95% de la masse totale du complexe, l'agent anesthésique est à une concentration comprise entre 5 et 60 mg d'agent anesthésique/kg d'eau, et **en ce que** les β-cyclodextrines utilisées pour la formulation de la solution anesthésique ont une teneur en humidité comprise entre 1 et 4%.

2. Solution anesthésique selon la revendication 1, dans laquelle la proportion de l'agent anesthésique dans le complexe d'inclusion est comprise entre 10 et 20%, et la proportion de β-cyclodextrines est comprise entre 80 et 90%.

3. Solution anesthésique selon l'une des revendications 1 ou 2, dans laquelle l'agent anesthésique est à une concentration comprise entre 10 et 40 mg d'agent anesthésique/kg d'eau.

4. Solution anesthésique selon l'une quelconque des revendications 1 à 3, **caractérisée en ce que** l'eau utilisée dans la solution est une bouillie de glace, où la glace est dans une proportion comprise entre 20 et 70% en poids par rapport au poids total du mélange d'eau et de glace.

5. Solution anesthésique selon l'une des revendications 1 à 3, **caractérisée en ce que** l'eau utilisée dans la solution est de l'eau avec de la glace pilée, où la proportion eau:glace en poids est comprise entre 1:1 et 1:3.

6. Utilisation d'une solution anesthésique selon les revendications 1 à 5 pour l'abattage de poissons.

7. Méthode d'abattage de poissons comprenant les étapes suivantes :
i) préparer une solution anesthésique selon les revendications 1 à 5, formulée dans une bouillie de glace à une température comprise entre -8°C et 0°C, ou dans de l'eau avec de la glace pilée à une température comprise entre -1°C et 0°C,
ii) immerger dans la solution préparée en i), au maximum, autant de kilogrammes de poissons que de kilogrammes de solution,
iii) maintenir les poissons immergés dans la solution préparée au point i) pendant une durée comprise entre 5 et 55 secondes, jusqu'à ce que les poissons soient étourdis,
iv) retirer les poissons étourdis de la solution,
v) introduire les poissons étourdis dans de l'eau avec de la glace dans une proportion eau:glace en poids comprise entre 1:1 et 1:3 et à une température comprise entre -1°C et 0°C dans une proportion, au maximum, d'autant de kilogrammes de poissons que de kilogrammes d'eau avec de la glace, jusqu'à ce que les poissons soient abattus.
